# FASCICULE DE BREVET EUROPEEN

(11) **EP 3 867 126 B1**
(45) Date de publication et mention de la délivrance du brevet: **13.09.2023**
(21) Numéro de dépôt: 19795289.8
(22) Date de dépôt: 01.10.2019
(51) Int. Cl.: B62D 15/02, G08G 1/16

(54) **PROCÉDÉ ET DISPOSITIF D'AIDE À LA CONDUITE D'UN VÉHICULE AUTOMOBILE EN PHASE DE RECUL**
VERFAHREN UND VORRICHTUNG ZUR UNTERSTÜTZUNG DES FAHRENS EINES KRAFTFAHRZEUGS WÄHREND DES RÜCKWÄRTSFAHRENS
METHOD AND DEVICE FOR ASSISTING WITH DRIVING A MOTOR VEHICLE DURING REVERSING

(30) Priorité: 17.10.2018 FR 1859587
(43) Date de publication de la demande: 25.08.2021
(73) Titulaire: PSA Automobiles SA, 78300 Poissy (FR)
(72) Inventeur: FEINGOLD, Laurent, 91400 SACLAY (FR); RIAT, Jean Christophe, 95870 BEZONS (FR); VRINAT, David, 92140 CLAMART (FR)
(86) Numéro de dépôt international: PCT/FR2019/052312
(87) Numéro de publication internationale: WO 2020/079340

(56) Documents cités:
- EP-A2- 2 743 133
- JP-A- 2001 307 298
- JP-A- 2018 148 530
- US-A1- 2018 050 639

## Description

### Domaine Technique

La présente invention se rapporte de manière générale à l'aide à la conduite des véhicules automobiles.

Elle concerne plus particulièrement un procédé et un dispositif d'aide à la conduite d'un véhicule automobile en phase de recul, c'est-à-dire durant une manoeuvre de recul (ou manoeuvre en marche-arrière) du véhicule.

### Arrière-plan Technologique

Certains véhicules récents, notamment dans le haut de gamme, présentent des versions équipées d'un système de rétro-vision numérique, en remplacement du rétroviseur intérieur (rétro-vision numérique dite « intérieure »), ou en remplacement des rétroviseurs extérieures (rétro-vision numérique dite « extérieure »). Pour la rétro-vision numérique intérieure, un tel système comprend une caméra numérique agencée à l'arrière du véhicule, de préférence en partie haute, pour acquérir dynamiquement des images de l'environnement extérieur à l'arrière du véhicule. Un calculateur embarqué est configuré pour recevoir et traiter les images acquises par la caméra numérique. Le système comprend aussi un écran d'affichage dédié, qui est agencé pour restituer au conducteur du véhicule des images fournies par le calculateur, et éventuellement des informations additionnelles relatives à l'environnement extérieur à l'arrière du véhicule, à partir des images acquises par la caméra numérique.

Cette technique de rétro-vision intérieure, également connue sous l'acronyme « RMR intérieure » (de l'anglais « *interior Rear view Mirer Replacement* »), vise à se substituer au traditionnel rétroviseur intérieur, en apportant notamment les avantages suivants : un champ de vision plus large et indépendant de la taille du pare-brise arrière et des panneaux de custode arrière ; bonnes performances de visibilité, quel que soit l'état de propreté du pare-brise arrière et même en conditions d'usage difficiles (en cas de pluie par exemple) ;; et le maintien de la visibilité arrière même en présence de trois passagers assis à l'arrière du véhicule et de bagages susceptibles d'occulter le cas échéant le pare-brise arrière.

Néanmoins, les nombreux avantages d'un tel système induisent une modification du comportement des conducteurs des véhicules qui en sont équipés. En particulier, de nombreux conducteurs n'appliquent pas les préconisations du code de la route pour la réalisation des manoeuvres de recul (ou manoeuvres en marche arrière), qui recommandent la vérification des rétroviseurs interne et externe(s), le mouvement de la tête du conducteur pour avoir une vision directe, etc... Des accidents pourraient résulter du non-respect de ces règles ou recommandations de sécurité.

### Art Antérieur

Le document DE 102 015 206 209 (correspondant au document WO 2016/162207) divulgue un dispositif de détermination de la vigilance du conducteur d'un véhicule automobile, suivant lequel une valeur de mesure représentant la vigilance d'un conducteur de véhicule est déterminée. Le procédé décrit comprend la détection de la direction de regard du conducteur, la détermination de la répartition de fréquence idéale de la direction du regard, et la détermination de la valeur de mesure en fonction d'un écart entre la répartition de fréquence idéale et la répartition de fréquence réelle de la direction du regard. Si le niveau de vigilance du conducteur reflété par cette valeur de mesure n'est pas suffisant, un message d'alerte peut être communiqué au conducteur. Le dispositif est capable de déterminer si le conducteur regarde différents éléments de l'habitacle pendant certaines durées, calculer la fréquence de ces regards, et en déduire un indicateur de la vigilance du conducteur.

Le document US 2018/0050639 décrit un système surveillant à la fois la position de la tête d'un conducteur et de ses yeux, et qui affiche les images sur un écran en fonction à la fois de cette position et des vues prises par une caméra de vision arrière.

### Résumé de l'invention

L'invention permet de superviser les actions du conducteur lors d'une manoeuvre de recul (ou manoeuvre du véhicule en marche arrière), afin de l'inciter à ne pas regarder uniquement l'écran du système de rétro-vision numérique.

A cet effet, un premier aspect de l'invention propose un procédé d'aide à la conduite d'un véhicule automobile en phase de recul, ledit véhicule comprenant :
- une unité de calcul ;
- un système de rétro-vision numérique ayant au moins une première caméra numérique agencée pour acquérir dynamiquement des images de l'environnement extérieur à l'arrière du véhicule, un calculateur configuré pour traiter les images acquises par la première caméra numérique, et un écran d'affichage agencé pour restituer au conducteur du véhicule des images fournies par le calculateur et/ou des informations relatives à l'environnement extérieur à l'arrière du véhicule à partir des images acquises par la première caméra numérique ; et, en outre
- un système de contrôle de vigilance du conducteur ayant au moins une seconde caméra numérique orientée vers le visage du conducteur pour acquérir des données d'oculométrie relatives au conducteur, et ayant en outre des moyens pour en déduire l'orientation du regard du conducteur,
ledit procédé comprenant les étapes suivantes, exécutées par l'unité de calcul durant une manoeuvre de recul du véhicule :
- détermination de l'orientation du regard du conducteur sur la base des données d'oculométrie relatives au conducteur, acquises dynamiquement par la seconde caméra numérique ; et,
- si le regard du conducteur est orienté seulement en direction de l'écran d'affichage du système de rétro-vision numérique pendant une durée supérieure à une durée seuil déterminée durant la manoeuvre de recul, affichage, sur l'écran d'affichage du système de rétro-vision numérique, d'une alerte visuelle invitant le conducteur à utiliser d'autres sources d'informations relatives à l'environnement extérieur à l'arrière du véhicule.

JP 2018/148530 divulgue les caractéristiques susmentionné dans un contexte de mouvement en avant.

Grâce à l'invention la procédure de conduite en phase de recul est supervisée, en ce sens qu'il est vérifié que le conducteur ne regarde pas uniquement l'écran du système de rétro-vision numérique. S'il est déterminé que l'attention visuelle du conducteur est portée sur l'écran de rétro-vision numérique au-delà d'une durée déterminée, typiquement 5 secondes, l'écran de rétro-vision numérique affiche une alerte au conducteur, lui faisant comprendre qu'il doit utiliser d'autres sources d'informations comme la vision directe ou via le ou les rétroviseurs extérieurs, comme il est recommandé dans le code de la route. L'invention prévoit de plus que l'unité de calcul soit en outre agencée pour échanger des données par l'intermediaire d'un système de communication inter-véhicules et que, lorsque l'unité de calcul reçoit, par le système de communication inter-véhicules, une information relative à la présence d'un objet dans l'environnement extérieur à l'arrière du véhicule mais en dehors du champ de vision de la caméra numérique du système de rétro-vision numérique, il affiche une alerte visuelle correspondante sur l'écran d'affichage du système de rétro-vision numérique.

Selon des modes de mises en oeuvre du procédé:
- le véhicule automobile comprend en outre un système d'aide au stationnement du véhicule comprenant au moins un capteur et/ou une caméra numérique pour détecter des évènements dans l'environnement extérieur à l'arrière du véhicule, dans lequel, lorsque le système d'aide au stationnement du véhicule détecte la présence d'un objet situé dans l'environnement extérieur à l'arrière du véhicule automobile mais en dehors du champ de vision de la caméra numérique du système de rétro-vision numérique, il est affiché une alerte visuelle correspondante sur l'écran d'affichage du système de rétro-vision numérique.
- la durée seuil déterminée est égale à quelques secondes, notamment comprise entre 3 et 8 secondes, par exemple égale à 5 secondes.
- une alerte visuelle affichée par l'écran d'affichage du système de rétro-vision numérique comprend un ou plusieurs des éléments compris dans l'ensemble constitué de : un pictogramme, un message texte, et un effet visuel comme un clignotement, une coloration ou une mise en surbrillance, par exemple.
- l'alerte visuelle invitant le conducteur à utiliser d'autres sources d'informations relatives à l'environnement extérieur à l'arrière du véhicule comprend une invitation à utiliser l'une au moins des sources d'informations comprises dans la liste constituée de l'utilisation des rétroviseurs extérieurs, l'utilisation d'informations fournies par un système d'aide au stationnement, la vision directe à travers les vitres du véhicule.
- un système automatique, tel qu'un système de freinage d'urgence par exemple, intervient sur la conduite du véhicule pour empêcher une action de conduite du conducteur si le regard du conducteur est orienté seulement en direction de l'écran d'affichage du système de rétro-vision numérique pendant une durée supérieure à la durée seuil déterminée durant la manoeuvre de recul.

Un deuxième aspect de l'invention concerne un dispositif d'aide à la conduite d'un véhicule automobile en phase de recul, comprenant des moyens logiciels pour exécuter toutes les étapes du procédé selon l'un des modes de mise en oeuvre du procédé selon le premier aspect ci-dessus.

Un troisième aspect de l'invention a encore pour objet un véhicule comprenant un dispositif selon le deuxième aspect. Un autre aspect, enfin, peut se rapporter à un produit programme d'ordinateur comprenant des instructions qui, lorsque le programme d'ordinateur est chargé dans la mémoire d'un ordinateur et est exécuté par un processeur dudit ordinateur, causent la mise en oeuvre par ledit ordinateur de toutes les étapes du procédé selon le premier aspect.

### Brève Description des Dessins

D'autres caractéristiques et avantages de l'invention apparaîtront encore à la lecture de la description qui va suivre. Celle-ci est purement illustrative et doit être lue en regard des dessins annexés sur lesquels :
[Fig. 1] est une représentation schématique du positionnement des moyens d'un système de rétro-vision numérique dans un véhicule automobile ;
[Fig. 2] est un schéma fonctionnel d'un dispositif selon des modes de réalisation de l'invention ; et,
[Fig. 3] est un diagramme d'étapes illustrant des modes de mises en oeuvre du procédé selon l'invention.

### Description détaillée de modes de réalisation

Dans la description de modes de réalisation qui va suivre et dans les Figures des dessins annexés, les mêmes éléments ou des éléments similaires portent les mêmes références numériques aux dessins.

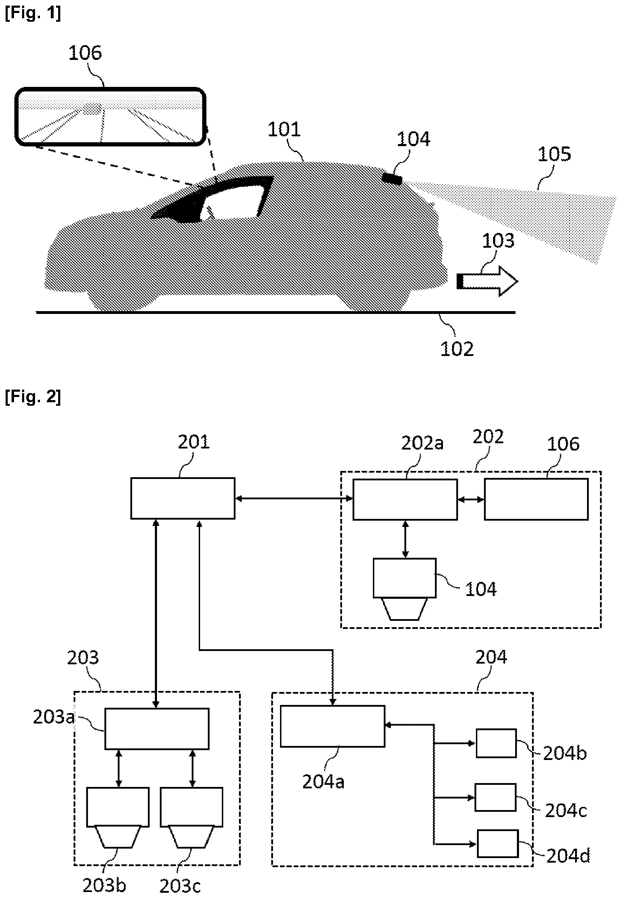

La **figure 1** montre schématiquement un véhicule automobile dans lequel le procédé d'aide à la conduite selon l'invention peut être mis en oeuvre.

Le véhicule automobile 101, circulant sur une route 102, est équipé d'un système de rétro-vision numérique 202. Le système 202 comprend au moins une caméra numérique 104, un écran d'affichage 106 et un calculateur 202a. Pour préserver la lisibilité de la figure, l'écran d'affichage 106 est représenté en dehors du véhicule automobile 101. Toutefois, dans la pratique, l'écran d'affichage 106 peut se situer n'importe où dans l'habitacle du véhicule automobile et dans le champ de vision du conducteur sans entraver sa bonne visibilité directe de la route, par exemple au niveau du combiné d'instruments, dans le centre de la planche de bord, ou à la place du rétroviseur intérieur en haut et au centre du parebrise avant, en étant tourné vers le visage et les yeux du conducteur.

La caméra numérique 104 est agencée pour acquérir dynamiquement des images de l'environnement extérieur à l'arrière du véhicule. Elle fournit ainsi des images particulièrement utiles au conducteur lorsque le véhicule se déplace en arrière c'est-à-dire lorsque le conducteur réalise une manoeuvre de recul, dite aussi manoeuvre en marche-arrière. A la figure 1, la flèche 103 illustre le sens du déplacement du véhicule dans une telle phase de conduite, et définit la direction arrière au sens de la présente description. Selon la technologie de la caméra, le champ de vision 105 de la caméra couvre typiquement une zone s'étendant jusqu'à environ 60 mètres vers l'arrière du véhicule.

Le calculateur 202a est configuré pour traiter les images acquises par la caméra numérique 104, et pour commander l'affichage de ces images sur l'écran d'affichage 106, soit directement soit après les avoir analysées et/ou traiter numériquement, le cas échéant. Par ailleurs, le calculateur peut commander l'affichage sur l'écran d'autres images et/ou informations, notamment relatives à l'environnement extérieur du véhicule, afin qu'elles soient affichées, par exemple, en surimpression des images acquises par la caméra numérique 104.

Avantageusement, le système de rétro-vision numérique 202 permet, entre-autres, de couvrir un champ de vision plus large qu'un rétroviseur classique, d'afficher une image lisible même en conditions de visibilité extérieur mauvaises (pluie, soleil intense...) et de s'affranchir, le cas échéant, de l'obstacle à la visibilité formé par des passagers et/ou bagages présents à l'arrière du véhicule.

En référence à la **figure 2****,** il va maintenant être décrit un exemple de dispositif au sein duquel le procédé d'aide à la conduite selon l'invention peut être mis en oeuvre. Le schéma-bloc de la figure 2 illustre les interactions fonctionnelles entre les différents éléments impliqués dans cette mise en oeuvre.

Le véhicule automobile 101 comprend une unité de calcul 201 avec un calculateur qui exécute un ou des éléments logiciels mettant en oeuvre les différentes étapes du procédé. Ce calculateur peut être, par exemple, le calculateur de contrôle du moteur qui assure le fonctionnement optimal du moteur du véhicule, ou le calculateur d'habitacle qui assure le fonctionnement des différents équipements de bord, ou encore un calculateur dédié. L'unité de calcul 201 communique avec différentes entités comprises dans le véhicule, ainsi qu'il va maintenant être décrit plus en détails.

En premier lieu, le système de rétro-vision numérique 202, tel qu'il a déjà été décrit plus haut en référence à la figure 1, fournit à l'unité de calcul 202 les informations en provenance de la caméra numérique 104. Les informations ainsi récupérées sont relatives à l'environnement extérieur à l'arrière du véhicule, et plus précisément dans le champ de vision 105 de la caméra 104 vers l'arrière du véhicule automobile 101.

En second lieu, l'unité de calcul 201 communique aussi avec un système de contrôle de vigilance du conducteur 203, aussi appelé système de mesure oculométrique. Dans l'exemple représenté, ce système comprend deux caméras numériques 203b et 203c. Néanmoins l'homme du métier appréciera que des telles mesures oculométriques peuvent être réalisées à partir d'une seule, ou au contraire à partir de plus de deux caméras numériques. Les caméras numériques 203b et 203c transmettent à une autre unité de calcul 203a des images qui, après analyse notamment des mouvements des yeux du conducteur (fonction dite de « *Eye-tracking* », en anglais) permettent de déduire et de suivre l'orientation du regard du conducteur en temps réel. Ces données sont transmises dynamiquement à l'unité de calcul 201.

Enfin, l'unité de calcul 201 communique avec un système d'aide au stationnement du véhicule 204 qui s'appuie sur des données fournies par un ensemble de capteurs pour aider le conducteur dans les manoeuvres de stationnement. Un ensemble de capteurs du système permet de détecter des évènements dans l'environnement extérieur du véhicule, et en particulier à l'arrière du véhicule. Un tel système peut être lui-même intégré à un système d'aide à la conduite (en anglais « *Advanced driver-assistance* systems » ou *ADAS*) plus général. Dans l'exemple représenté, une unité de calcul 204a récupère et traite des données en provenance de capteurs 204b, 204c et 204d pour localiser le véhicule par rapport aux différents éléments présents dans son environnement extérieur. L'homme du métier appréciera que la fonction du système d'aide au stationnement 204 peut être réalisée, dans différents modes de réalisation, à partir de données obtenues par un ou plusieurs capteurs et/ou une ou plusieurs caméras numériques. Finalement, le système d'aide au stationnement 204 transmet dynamiquement, à l'unité de calcul 201, les données obtenues à partir de son (ou ses) capteur(s) et/ou de sa (ou ses) caméra(s).

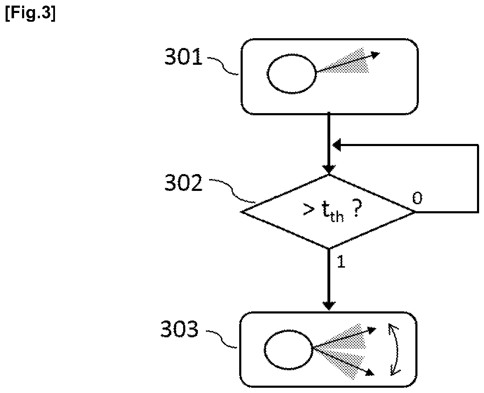

En référence à la **figure 3****,** il va maintenant être décrit un diagramme d'étapes illustrant les différentes étapes exécutées par l'unité de calcul 201 pour réaliser le procédé d'aide à la conduite d'un véhicule automobile en phase de recul selon l'invention. Le procédé est initié dès que le véhicule automobile se trouve manoeuvré dans une phase de recul c'est-à-dire dans la direction arrière, par exemple en réponse à l'enclenchement de la marche arrière au niveau du boîtier de sélection de vitesses.

A l'étape 301 on détermine l'orientation du regard du conducteur sur la base des données d'oculométrie relatives au conducteur, qui sont acquises dynamiquement par au moins une caméra numérique du système 203 de contrôle de vigilance du conducteur. L'orientation du regard du conducteur est ainsi déterminée en temps réelle par l'unité de calcul 201.

On vérifie à l'étape 302 la durée pendant laquelle le regard du conducteur reste orienté vers l'écran d'affichage 106 du système de rétro-vision numérique 202, c'est-à-dire est orienté sans interruption vers cet écran. L'étape suivante du procédé n'est réalisée que si, durant une manoeuvre de recul, le regard du conducteur est orienté seulement en direction de l'écran d'affichage du système de rétro-vision numérique pendant une durée supérieure à une durée seuil déterminée tₜₕ, de quelques secondes. La durée seuil tₜₕ peut être, dans des modes de mise en oeuvre du procédé, comprise entre 3 et 8 secondes. Par exemple, elle peut être égale à 5 secondes.

Enfin, l'étape 303 consiste à afficher, sur l'écran d'affichage 106 du système de rétro-vision numérique 202, une alerte visuelle invitant le conducteur à utiliser d'autres sources d'informations relatives à l'environnement extérieur à l'arrière du véhicule. Par exemple, le conducteur peut être invité à utiliser les rétroviseurs extérieurs, à utiliser les informations fournies par le système d'aide au stationnement, ou à observer l'environnement extérieur à l'arrière du véhicule par vision directe à travers les vitres du véhicule. De cette façon, le conducteur est incité à regarder dans toutes les directions utiles, ce qui réduite le risque de percuter un obstacle et améliore la sécurité du véhicule et la sécurité des tiers (autres véhicule, piétons, cyclistes, propriétaire de mobilier urbain, etc.).

En outre, dans un mode de mise en oeuvre particulier du procédé, l'affichage d'une alerte peut aussi être déclenchée consécutivement à la détection, par un capteur et/ou une caméra numérique du système 204 d'aide au stationnement, d'un objet situé dans l'environnement extérieur à l'arrière du véhicule automobile mais en dehors du champ de vision de la caméra numérique du système de rétro-vision numérique.

Les différentes alertes visuelles susceptibles d'être affichées, en plus des images acquises par la caméra numériques 104, par l'écran d'affichage 106 du système de rétro-vision numérique, sont destinées à transmettre des informations ou indications au conducteur. En ce sens, il peut s'agir, par exemple, de pictogrammes, de messages texte ou d'effets visuels montrés sur l'écran 106 tels qu'un clignotement, une coloration ou une mise en surbrillance d'une portion de l'écran, d'un texte affiché, d'un pictogramme, etc. De cette façon, l'écran peut en particulier permettre d'indiquer clairement au chauffeur où porter son regard et son attention, par exemple en réponse à et en relation avec la détection d'un objet dans l'environnement extérieur du véhicule (à l'arrière ou à l'avant).

Certains véhicules automobiles sont aujourd'hui équipés de la technologie appelée « *Car-to-X* ». Il s'agit d'un système connu de communication inter-véhicules (no-représenté sur les figures) qui peut, entre-autres, permettre à des véhicules de se localiser respectivement dans l'espace. Ainsi, dans un mode de mise en oeuvre particulier du procédé, l'unité de calcul 201 du véhicule automobile peut être agencée pour échanger des données par l'intermédiaire d'un système de communication inter-véhicules de type « *Car-to-*X ». Dans ce cas, et additionnellement aux informations obtenues le cas échéant du système d'aide au stationnement, l'unité de calcul peut recevoir de ce système, une information relative à la présence d'un objet dans l'environnement extérieur à l'arrière du véhicule mais en dehors du champ de vision de la caméra numérique du système de rétro-vision numérique. Lorsque cela se produit, le conducteur peut aussi être alerté visuellement par l'intermédiaire de l'écran d'affichage du système de rétro-vision numérique. Avantageusement, le conducteur peut être incité à regarder précisément dans la direction où le véhicule est susceptible de rencontrer un obstacle, que ce soit à l'arrière ou à l'avant du véhicule.

Enfin, dans un mode de mise en oeuvre particulier, lorsque l'étape 302 confirme l'orientation du regard du conducteur en direction de l'écran 106 pendant une durée supérieure à tₜₕ, un système automatique, tel qu'un système de freinage d'urgence par exemple, peut intervenir directement sur la conduite du véhicule. En particulier, ce système peut déclencher un freinage d'urgence afin d'éviter un choc avec un objet qu'un réflexe humain n'aurait pas pu empêcher.

Dans les revendications, le terme "comprendre" ou "comporter" n'exclut pas d'autres éléments ou d'autres étapes. Un seul processeur ou plusieurs autres unités peuvent être utilisées pour mettre en oeuvre l'invention. Les signes de référence ne sauraient être compris comme limitant la portée de l'invention.

## Revendications

1. Procédé d'aide à la conduite d'un véhicule automobile en phase de recul, ledit véhicule comprenant :
- une unité de calcul (201) ;
- un système de rétro-vision numérique (202) ayant au moins une première caméra numérique (104) agencée pour acquérir dynamiquement des images de l'environnement extérieur à l'arrière du véhicule, un calculateur (202a) configuré pour traiter les images acquises par la première caméra numérique, et un écran d'affichage (106) agencé pour restituer au conducteur du véhicule des images fournies par le calculateur et/ou des informations relatives à l'environnement extérieur à l'arrière du véhicule à partir des images acquises par la première caméra numérique ; et, en outre
- un système de contrôle de vigilance du conducteur (203) ayant au moins une seconde caméra numérique (203b) orientée vers le visage du conducteur pour acquérir des données d'oculométrie relatives au conducteur, et ayant en outre des moyens (203a) pour en déduire l'orientation du regard du conducteur,
ledit procédé comprenant les étapes suivantes, exécutées par l'unité de calcul durant une manoeuvre de recul du véhicule :
- détermination de l'orientation du regard du conducteur (301) sur la base des données d'oculométrie relatives au conducteur, acquises dynamiquement par la seconde caméra numérique ; et,
- si le regard du conducteur est orienté seulement en direction de l'écran d'affichage du système de rétro-vision numérique pendant une durée supérieure à une durée seuil déterminée (tₜₕ) durant la manoeuvre de recul, affichage (303), sur l'écran d'affichage du système de rétro-vision numérique, d'une alerte visuelle invitant le conducteur à utiliser d'autres sources d'informations relatives à l'environnement extérieur à l'arrière du véhicule ; l'unité de calcul est en outre agencée pour échanger des données par l'intermédiaire d'un système de communication inter-véhicules et dans lequel, lorsque l'unité de calcul reçoit, par le système de communication inter-véhicules, une information relative à la présence d'un objet dans l'environnement extérieur à l'arrière du véhicule mais en dehors du champ de vision de la caméra numérique du système de rétro-vision numérique, il est affiché une alerte visuelle correspondante sur l'écran d'affichage du système de rétro-vision numérique.

2. Procédé selon la revendication 1, dans lequel le véhicule automobile comprend en outre un système d'aide au stationnement du véhicule comprenant au moins un capteur et/ou une caméra numérique pour détecter des évènements dans l'environnement extérieur à l'arrière du véhicule, dans lequel, lorsque le système d'aide au stationnement du véhicule détecte la présence d'un objet situé dans l'environnement extérieur à l'arrière du véhicule automobile mais en dehors du champ de vision de la caméra numérique du système de rétro-vision numérique, il est affiché une alerte visuelle correspondante sur l'écran d'affichage du système de rétro-vision numérique.

3. Procédé selon l'une quelconque des revendications 1 à 2, dans lequel la durée seuil déterminée (tₜₕ) est égale à quelques secondes, notamment comprise entre 3 et 8 secondes, par exemple égale à 5 secondes.

4. Procédé selon l'une quelconque des revendications 1 à 3, dans lequel une alerte visuelle affichée par l'écran d'affichage du système de rétro-vision numérique comprend un ou plusieurs des éléments compris dans l'ensemble constitué de : un pictogramme, un message texte, et un effet visuel.

5. Procédé selon l'une quelconque des revendications 1 à 4, dans lequel l'alerte visuelle invitant le conducteur à utiliser d'autres sources d'informations relatives à l'environnement extérieur à l'arrière du véhicule comprend une invitation à utiliser l'une au moins des sources d'informations comprises dans la liste constituée de l'utilisation d'un plusieurs rétroviseurs extérieurs, l'utilisation d'informations fournies par un système d'aide au stationnement, la vision directe à travers les vitres du véhicule.

6. Procédé selon l'une quelconque des revendications 1 à 5, dans lequel un système automatique, comme par exemple un système de freinage d'urgence, intervient sur la conduite du véhicule pour empêcher une action de conduite du conducteur si le regard du conducteur est orienté seulement en direction de l'écran d'affichage du système de rétro-vision numérique pendant une durée supérieure à la durée seuil déterminée (tₜₕ) durant la manoeuvre de recul.

7. Dispositif d'aide à la conduite d'un véhicule automobile en phase de recul, comprenant des moyens logiciels pour exécuter toutes les étapes du procédé selon l'une quelconque des revendications 1 à 6.

8. Véhicule automobile comprenant un dispositif selon la revendication 7.

9. Produit programme d'ordinateur comprenant des instructions qui, lorsque le programme d'ordinateur est chargé dans la mémoire d'un ordinateur et est exécuté par un processeur dudit ordinateur, causent la mise en oeuvre par l'ordinateur de toutes les étapes du procédé selon l'une quelconque des revendications 1 à 5.

## Patentansprüche

1. Verfahren zur Unterstützung des Fahrens eines Kraftfahrzeugs in der Rückwärtsphase, wobei das Fahrzeug Folgendes umfasst:
- eine Berechnungseinheit (201);
- ein digitales Rücksichtsystem (202) mit mindestens einer ersten Digitalkamera (104), die so angeordnet ist, dass sie dynamisch Bilder der Außenumgebung am Heck des Fahrzeugs erfasst, und einem Computer (202a), der so konfiguriert ist, dass er die von der ersten erfassten Bilder verarbeitet Digitalkamera und einen Anzeigebildschirm (106), der dafür ausgelegt ist, dem Fahrer des Fahrzeugs vom Computer gelieferte Bilder und/oder Informationen über die äußere Umgebung am Heck des Fahrzeugs aus den von der ersten Digitalkamera erfassten Bildern wiederzugeben; und zusätzlich
- ein Kontrollsystem für die Aufmerksamkeit des Fahrers (203), das mindestens eine zweite Digitalkamera (203b) aufweist, die auf das Gesicht des Fahrers ausgerichtet ist, um Blickverfolgungsdaten bezüglich des Fahrers zu erfassen, und weiterhin Mittel (203a) zum Ableiten der Orientierung des Fahrers aufweist Blick,
wobei das Verfahren die folgenden Schritte umfasst, die von der Berechnungseinheit während eines Rückwärtsmanövers des Fahrzeugs ausgeführt werden:
- Ermitteln der Blickrichtung des Fahrers (301) anhand von Eye-Tracking-Daten des Fahrers, die von der zweiten Digitalkamera dynamisch erfasst werden; Und,
- wenn der Blick des Fahrers während des Rückfahrmanövers für eine Dauer größer als eine bestimmte Schwellendauer (tₜₕ) nur in Richtung des Anzeigebildschirms des digitalen Rückblicksystems gerichtet ist, Anzeige (303) auf dem digitalen Rückblick Systemanzeigebildschirm, eine visuelle Warnung, die den Fahrer auffordert, andere Informationsquellen in Bezug auf die Außenumgebung am Heck des Fahrzeugs zu nutzen; Die Berechnungseinheit ist außerdem so angeordnet, dass sie Daten über ein Kommunikationssystem zwischen Fahrzeugen austauscht, und wenn die Berechnungseinheit über das Kommunikationssystem zwischen Fahrzeugen Informationen über das Vorhandensein eines Objekts in der Außenumgebung am Heck empfängt Befindet sich das Fahrzeug jedoch außerhalb des Sichtfelds der Digitalkamera des digitalen Rückfahrsystems, wird auf dem Bildschirm des digitalen Rückfahrsystems eine entsprechende optische Warnung angezeigt.

2. Verfahren nach Anspruch 1, bei dem das Kraftfahrzeug weiterhin ein Fahrzeugparkassistenzsystem umfasst, das mindestens einen Sensor und/oder eine Digitalkamera zur Erkennung von Ereignissen in der Außenumgebung am Heck des Fahrzeugs umfasst. Wenn das Parkassistenzsystem des Fahrzeugs die Anwesenheit eines Objekts erkennt, das sich in der äußeren Umgebung am Heck des Kraftfahrzeugs befindet, jedoch außerhalb des Sichtfelds der Digitalkamera des Rückfahrsystems - Digital Vision, erfolgt eine entsprechende visuelle Warnung auf dem Bildschirm des digitalen Rückfahrsystems angezeigt.

3. Verfahren nach einem der Ansprüche 1 bis 2, bei dem die bestimmte Schwellenwertdauer (tₜₕ) einige Sekunden beträgt, insbesondere zwischen 3 und 8 Sekunden, beispielsweise 5 Sekunden.

4. Verfahren nach einem der Ansprüche 1 bis 3, bei dem eine visuelle Warnung, die auf dem Bildschirm des digitalen Rückfahrsystems angezeigt wird, eines oder mehrere der in der Gruppe enthaltenen Elemente umfasst, bestehend aus: einem Piktogramm, einer Textnachricht und einen visuellen Effekt.

5. Verfahren nach einem der Ansprüche 1 bis 4, bei dem die visuelle Warnung, die den Fahrer auffordert, andere Informationsquellen bezüglich der Außenumgebung am Heck des Fahrzeugs zu nutzen, eine Aufforderung zur Nutzung der mindestens einen der Quellen umfasst der in der Liste enthaltenen Informationen, bestehend aus der Verwendung eines oder mehrerer Außenspiegel, der Verwendung von Informationen eines Parkassistenzsystems und der direkten Sicht durch die Fenster des Fahrzeugs.

6. Verfahren nach einem der Ansprüche 1 bis 5, bei dem ein automatisches System, wie beispielsweise ein Notbremssystem, in den Fahrbetrieb des Fahrzeugs eingreift, um eine Fahrhandlung des Fahrers zu verhindern, wenn der Blick des Fahrers nur gerichtet ist in Richtung des Anzeigebildschirms des digitalen Rückfahrsystems für eine Dauer größer als die ermittelte Schwellendauer (tₜₕ) während des Rückwärtsmanövers.

7. Vorrichtung zur Unterstützung des Fahrens eines Kraftfahrzeugs in der Rückwärtsphase, umfassend Softwaremittel zur Ausführung aller Schritte des Verfahrens nach einem der Ansprüche 1 bis 6.

8. Kraftfahrzeug umfassend eine Vorrichtung nach Anspruch 7.

9. Computerprogrammprodukt, das Anweisungen umfasst, die, wenn das Computerprogramm in den Speicher eines Computers geladen und von einem Prozessor des Computers ausgeführt wird, den Computer veranlassen, alle Schritte des Verfahrens nach einem der Ansprüche 1 bis auszuführen 5.

## Claims

1. Method for assisting the driving of a motor vehicle in the reversing phase, said vehicle comprising:
- a calculation unit (201);
- a digital rear-vision system (202) having at least a first digital camera (104) arranged to dynamically acquire images of the external environment at the rear of the vehicle, a computer (202a) configured to process the acquired images by the first digital camera, and a display screen (106) arranged to restore to the driver of the vehicle images supplied by the computer and/or information relating to the external environment at the rear of the vehicle from the images acquired by the first digital camera; and, in addition
- a driver vigilance control system (203) having at least a second digital camera (203b) oriented towards the face of the driver to acquire eye tracking data relating to the driver, and further having means (203a) for deduce the orientation of the driver's gaze,
said method comprising the following steps, executed by the calculation unit during a reversing maneuver of the vehicle:
- determination of the orientation of the driver's gaze (301) on the basis of eye tracking data relating to the driver, acquired dynamically by the second digital camera; And,
- if the driver's gaze is oriented only in the direction of the display screen of the digital rear-vision system for a duration greater than a determined threshold duration (tₜₕ) during the reversing maneuver, display (303), on the digital rear-vision system display screen, a visual alert inviting the driver to use other sources of information relating to the outside environment at the rear of the vehicle;
the calculation unit is also arranged to exchange data via an inter-vehicle communication system and in which, when the calculation unit receives, via the inter-vehicle communication system, information relating to the presence of an object in the external environment at the rear of the vehicle but outside the field of vision of the digital camera of the digital rear-view system, a corresponding visual alert is displayed on the screen of digital rear-view system display.

2. Method according to claim 1, in which the motor vehicle further comprises a vehicle parking assistance system comprising at least one sensor and/or a digital camera for detecting events in the external environment at the rear of the vehicle. vehicle, in which, when the parking assistance system of the vehicle detects the presence of an object located in the external environment at the rear of the motor vehicle but outside the field of vision of the digital camera of the rear view system - digital vision, a corresponding visual alert is displayed on the display screen of the digital rear-vision system.

3. Method according to any one of claims 1 to 2, in which the determined threshold duration (tₜₕ) is equal to a few seconds, in particular between 3 and 8 seconds, for example equal to 5 seconds.

4. Method according to any one of claims 1 to 3, in which a visual alert displayed by the display screen of the digital rear-vision system comprises one or more of the elements included in the set consisting of: a pictogram , a text message, and a visual effect.

5. Method according to any one of claims 1 to 4, in which the visual alert inviting the driver to use other sources of information relating to the external environment at the rear of the vehicle comprises an invitation to use the at least one of the sources of information included in the list consisting of the use of one or more exterior mirrors, the use of information provided by a parking assistance system, direct vision through the windows of the vehicle .

6. Method according to any one of claims 1 to 5, in which an automatic system, such as for example an emergency braking system, intervenes on the driving of the vehicle to prevent a driving action by the driver if the driver's gaze is oriented only in the direction of the display screen of the digital rear-vision system for a duration greater than the determined threshold duration (tₜₕ) during the reversing manoeuvre.

7. Device for assisting the driving of a motor vehicle in the reversing phase, comprising software means for executing all the steps of the method according to any one of claims 1 to 6.

8. Motor vehicle comprising a device according to claim 7.

9. Computer program product comprising instructions which, when the computer program is loaded into the memory of a computer and is executed by a processor of said computer, cause the computer to perform all the steps of the method according to any one of claims 1 to 5.
